# EUROPEAN PATENT SPECIFICATION

(11) **EP 3 634 501 B1**
(45) Date of publication and mention of the grant of the patent: **20.09.2023**
(21) Application number: 18732925.5
(22) Date of filing: 04.06.2018
(51) Int. Cl.: A61L 2/07, A61L 2/18, A61L 2/26, B65G 19/14

(54) **STERILIZING MACHINE FOR LOOSE PRODUCTS**
STERILISIERMASCHINE FÜR LOSE PRODUKTE
MACHINE DE STÉRILISATION POUR PRODUITS EN VRAC

(30) Priority: 05.06.2017 IT 201700061201
(43) Date of publication of application: 15.04.2020
(73) Proprietor: Icos Pharma S.p.A., 33080 Zoppola (IT)
(72) Inventor: BENI, Stefano, 33170 Pordenone (IT)
(74) Representative: Petraz, Gilberto Luigi
(86) International application number: PCT/IT2018/050098
(87) International publication number: WO 2018/225105

(56) References cited:
- EP-A1- 0 672 426
- EP-A1- 3 064 226
- EP-A2- 0 510 276
- EP-A2- 0 588 145
- EP-A2- 1 920 853
- WO-A1-00/04935
- WO-A1-2007/014312
- WO-A1-2015/150581
- DE-A1- 3 841 930
- DE-C1- 3 938 546
- JP-A- H11 290 061
- US-A- 5 397 535
- US-A- 5 656 248

## Description

### FIELD OF THE INVENTION

The present invention concerns a sterilizing machine for loose products, for example a sterilizing machine with a rotating drum which can be used, in particular, in the hospital, pharmaceutical or food field, or in the industrial sector for the production of loose products.

The loose products can be, for example, closing stoppers for test tubes for laboratory analysis, closing stoppers for vials of drugs, or small instruments or objects used in operating theaters or laboratories, or other.

### BACKGROUND OF THE INVENTION

It is known that in the hospital or pharmaceutical field, or other zones, such as the food industry, it is necessary to use sterile products to prevent contamination or alteration of substances with which they come into contact, or to avoid introducing bacteria, germs, other corpuscles or potentially pathogenic or harmful agents into environments where their total absence is required.

Among such sterile products, in all the above mentioned fields, loose products, even small ones, are normally present, such as, for example, stoppers for closing test tubes intended to contain substances to be subjected to laboratory analysis, or small instruments or objects used in operating theaters or laboratories.

The loose products can already be sterile when they are produced and sold in packages which are themselves sterile, or are sterilized before each use and reused several times during their working life.

In some cases the loose products therefore require to be sterilized. Some examples of machines or devices for sterilizing objects are described for example in documents DE-A-3841930, WO-A-2015150581 and EP-A-3064226.

In other documents, for example documents US-A-5397535, EP-A-0588145 and WO-A-2007014312 operations to sterilize or disinfect objects are described in various ways, also used in the healthcare sector or suchlike.

Moreover, in document EP-A-1920853 a machine for cleaning and drying objects is described, while in document JP-A-H11290061 a heat treatment process of a pneumatic transport pipe is described.

It is also known to sterilize these loose products in sterilization machines provided with a sealed chamber in which a rotating drum receives the products to be sterilized from a so-called "dirty" loading side, sterilizes them during a treatment cycle normally comprised between 1 and 5 hours, and unloads them from a so-called "sterile" unloading side, where they are generally packaged in sterile packs.

Rotating drums are known, partitioned into sectors, each containing a desired quantity of loose products to be poured into the individual packs after sterilization.

As is known, the loose products are generally loaded into the treatment chamber provided with the drum by means of a loading zone situated above the treatment chamber. A containing sector of the rotating drum faces toward the loading zone on each occasion, in such a way as to introduce a correct quantity of loose products into the containing sector.

The loading of the loose products through the loading zone can be done manually, or by means of a pneumatic system which sucks the products from a basket and sends them to a metering cylinder located in correspondence with the loading zone of the treatment chamber.

One disadvantage of known sterilizing apparatuses or machines, including the various machines and apparatuses cited in the above documents, is that known loading systems often prove to be not very effective, both in terms of productivity and efficiency, moreover, in general, the loose products inside the baskets are always in a static or stationary situation and therefore there is a risk that they can undesirably accumulate with each other.

Furthermore, if the loose products are, for example, coated with Teflon, or suchlike, there is a real risk that the loose products can stick to each other in the containing baskets.

Another disadvantage, moreover, is that often known sterilizing machines are not sufficiently automated and therefore require frequent interventions by the operators, both in the normal steps of loading the loose products, and also, for example, for steps to control the treatment process which is carried out in the various containing sectors of the drum.

A control which is carried out at the end of the treatment cycle, for example, can provide for an operator to open a door of the treatment chamber to verify that possible residual loose products have not remained undesirably in one of the containing sectors of the drum, so as to prevent the residual and treated loose products from mixing with loose products to be treated and/or with loose products of different types.

The problems and disadvantages cited above in particular for the loading operations of sterilizing machines with a rotating drum could also be similar in the case of sterilizing machines with a treatment chamber in which there is no rotating drum, but in which, for example, the loose products to be treated are housed in other containing elements.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to perfect a sterilizing machine which can overcome at least one of the disadvantages of the prior art.

One purpose of the present invention is therefore to provide a sterilizing machine which is provided with an effective and automated treatment cycle, in particular with regard to the loading system of the loose products to be treated.

Another purpose of the present invention is to provide a sterilizing machine which is equipped with a system for loading the loose products to be treated which makes the loading operations of the loose products into the treatment chamber extremely fluid and precise.

Another purpose of the present invention is to provide a sterilizing machine which keeps the loose products loaded moving, and prevents excessive accumulation and grouping of loose products during loading operations, which would cause a reduction in the overall efficiency of the machine.

Another purpose of the present invention is to perfect an efficient and precise method to sterilize loose products.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a sterilizing machine for loose products according to the present invention comprises a treatment chamber provided with a loading zone for the loose products to be treated.

In some embodiments, the sterilizing machine comprises, upstream of the treatment chamber, a container for loading the loose products provided with a loading side and an unloading side of the loose products and located in rotation by means of a drive motor around an axis that passes through the loading side and the unloading side; at least one helicoidal guide with several spirals, positioned inside the container and configured to move and guide the loose products from the loading side to the unloading side; at least a pipe for sending the loose products from the container to the treatment chamber; and at least one pneumatic device positioned on the unloading side of the container and configured to introduce the loose products unloaded from the container into the pipe and hence toward the loading zone of the treatment chamber.

The container can be a rotatable drum provided with a cylindrical casing comprising an internal chamber where the helicoidal guide develops along the axis of rotation.

The container can comprise, on the loading side, a first tubular element communicating with a hopper for loading the loose products and, on the unloading side, a second tubular element to transfer the loose products toward the pneumatic device.

The container can comprise truncated cone parts to connect with the tubular elements.

The first tubular element can be integrated with the rotating container and can be connected to the loading hopper by means of a rotatable joint.

The second tubular element can be integrated with the rotating container and can be connected, by means of a rotatable joint, to another tubular element to transfer the loose products.

The machine can comprise a valve for opening and closing the passage of the loose products positioned downstream of the unloading side of the container and upstream of the pneumatic device.

The machine can comprise a metering device to meter the loose products, positioned downstream of the loading container.

The metering device can be positioned upstream of a connector connecting with the pneumatic device for sending the loose products to the treatment chamber.

The machine can comprise a control device inside the treatment chamber.

The machine can comprise at least a control unit connected at least to the opening and closing valve, to the metering device and to the control device.

The machine can also comprise a frame provided with rolling elements to position and support the rotatable container.

The loading container can have an inclination from the top downward from the loading side to the unloading side.

The present invention also concerns a method for sterilizing loose products, carried out in a sterilizing machine for loose products. According to a characteristic aspect of the invention the method comprises loading the loose products to be treated into a loading container provided with a loading side and an unloading side and rotatable by means of a drive motor around an axis that passes through the loading side and the unloading side; guiding and moving the loose products by means of at least a helicoidal guide with several spirals positioned inside the container and configured to guide and move the loose products from the loading side to the unloading side; and sending the loose products toward a loading zone of a treatment chamber of the sterilizing machine once the loose products have exited from the unloading side of the loading container; the sending is carried out by means of a pneumatic device that introduces the loose products exiting from the unloading side into a pipe provided between the loading side of the treatment chamber and the unloading side of the container.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:

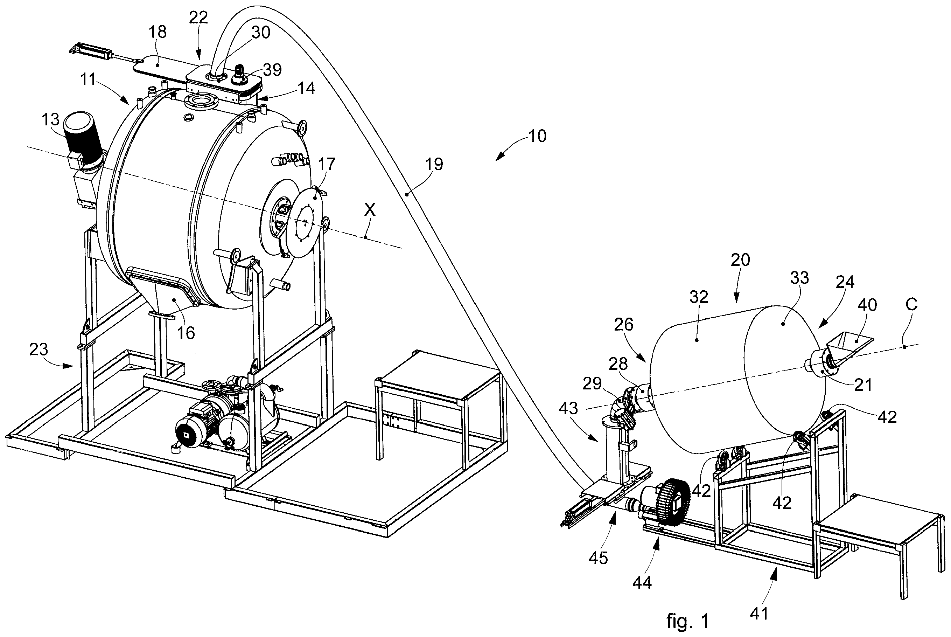

- fig. 1 is a three dimensional view of a sterilizing machine according to the present invention;

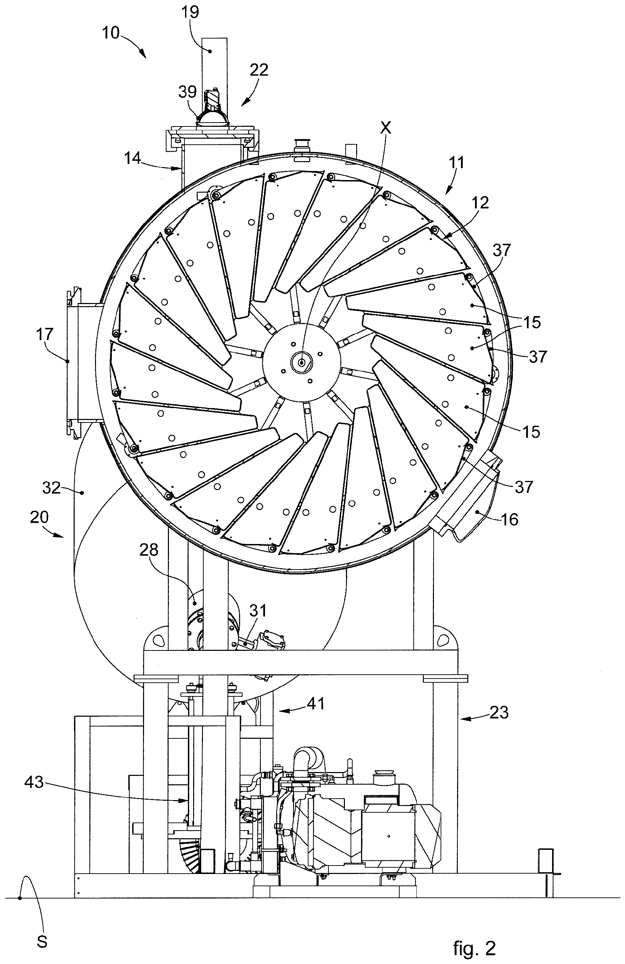

- fig. 2 is a cross section view of the sterilizing machine in fig. 1;

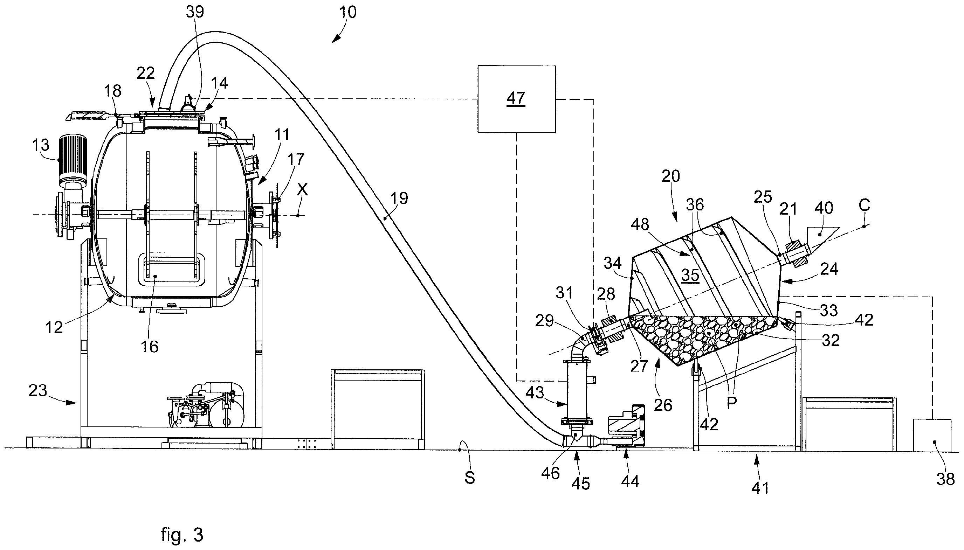

- fig. 3 is a view of the sterilizing machine in lateral elevation and in section.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. We must also clarify that the phraseology and terminology used here is for the purposes of description only, and cannot be considered as limitative.

With reference to the attached drawings, a sterilizing machine 10 according to the invention comprises a treatment chamber 11 in which the loose products P to be treated are housed.

By way of non-restrictive example, reference will be made in the following description to a sterilizing machine 10 in whose treatment chamber 11 a rotatable drum 12 is housed, however, the description could refer to a different type of sterilizing machine comprising a treatment chamber 11 for the loose products P without a rotatable drum 12, but provided, for example, with other containing elements of the loose products P to be treated.

The drum 12 can rotate around an axis X and is made to rotate by suitable drive means 13.

The drum 12 is substantially cylindrical in shape and is subdivided into a series of containing sectors 15, able to contain a determinate quantity of loose products P to be treated.

The containing sectors 15 typically have a pyramidal and widened shape proceeding from the center to the periphery of the drum 12.

In proximity to the periphery, each of the containing sectors 15 comprises a door 37, which can be selectively closed or opened to allow the loading of the loose products P to be treated inside the corresponding containing sector 15 and the unloading of the treated loose products P from the containing sector 15.

The treatment chamber 11 comprises a loading zone 14 of the loose products P to be treated inside each of the containing sectors 15.

In general, the loading zone 14, as shown, is positioned on an upper part of the treatment chamber 11.

The loading zone 14 comprises a loading aperture 30 which can be opened and closed by means of a valve 22, for example a guillotine valve, comprising a shutter 18 which can be translated from an open position to a closed position, or vice versa.

The present sterilizing machine 10 also comprises a control device 39 to control the drum 12, in particular the corresponding containing sectors 15.

The control device 39 can be located in correspondence with the loading aperture 30, so as to verify the correct loading of the containing sector 15 positioned in correspondence with the loading zone 14.

The control device 39, preferably functioning automatically, can be, for example, a digital camera, an optical sensor or suchlike, and will be associated with a control unit 47 of the sterilizing machine 10.

The control device 39 can be activated to verify at least the state of the containing sector 15 located in proximity to the loading zone 14.

As we said, therefore, by means of the control device 39, it is possible to verify the correct presence of loose products P to be treated in the containing sector 15 located in correspondence with the loading zone 14, but also to verify that at the end of the treatment cycle there are no loose products P which, undesirably, have remained in the containing sectors 15, or other.

If any possible loose products P have been undesirably trapped in one of the containing sectors 15, it is possible to send an alarm and provide to open a door 17, by means of which the various containing sectors 15 of the drum 12 can be accessed.

The treatment chamber 11 also comprises an unloading aperture 16, from which, on each occasion, the loose products P treated in each containing sector 15 exit.

The unloading aperture 16 is preferably positioned in a lower part of the sterilizing machine 10, so that the treated loose products P coming from a determinate containing sector 15 pass through the aperture 16 due to gravity.

The treatment chamber 11 comprises a support frame 23, by means of which, preferably, it is placed at a certain height with respect to the ground, or support plane, S.

Upstream of the treatment chamber 11 a container 20 is positioned to load the loose products P, comprising a loading side 24 and an unloading side 26.

The container 20 is made to rotate by a drive motor 38 and around an axis C, which passes through the loading side 24 and the unloading side 26, so as to keep the loose products P continuously in motion and allow a correct movement thereof from the loading side 24 to the unloading side 26. The axis C can be the longitudinal central axis of the container 20.

Inside it, the container 20 also comprises a helicoidal guide 48 with several spirals 36. The helicoidal guide 48 has the function of guiding the loose products P and moving them uniformly from the loading side 24 to the unloading side 26, during the rotation of the container 20 around the axis C.

The container 20 can be in the form of a rotatable drum provided with a cylindrical casing 32 comprising an internal chamber 35 where the helicoidal guide 48 develops along the axis of rotation C.

The container 20 can comprise, on the loading side 24, a first tubular element 25 communicating with a loading hopper 40, and, on the unloading side 26, another tubular element 27.

The drive motor 38 could be connected in a known manner and by suitable transmission elements to at least one of the tubular elements 25 and 27, protruding respectively from the loading side 24 and from the unloading side 26 of the container 20.

Alternatively, if the container 20 comprises the cylindrical shaped casing 32, as shown by way of example in the case here, the drive motor 38 could be connected to a toothed element or suchlike provided around the casing 32.

The casing 32, if substantially cylindrical in shape, can comprise truncated cone parts 33 and 34 located in correspondence with the loading side 24 and the unloading side 26, so as to provide an adequate connection with the tubular elements 25 and 27.

The container 20 will be supported by a suitable support frame 41.

The support frame 41 is able to confer on the container 20 an inclination from the top downwards from the loading side 24 to the unloading side 26, so as to promote the passage of the loose products P from the loading side 24 to the unloading side 26.

The support framework 41 provides, as shown, rolling elements 42, such as wheels or suchlike, to support the rotating container 20, in particular its casing 32.

The tubular element 25 which protrudes from the loading side 24 and which is integral with the rotating container 20 can be connected to the loading hopper 40 by means of a rotatable joint 21.

The tubular element 27 which protrudes from the unloading side 26 of the container 20 and which is integral with the rotating container 20 can be connected to another tubular element 29 to transfer the loose products P by means of another rotatable joint 28.

Upstream of the tubular element 29 and downstream of the unloading side 26 of the container 20 a valve 31 is provided to open and close the passage of the loose products P.

Inside the casing 32 the chamber 35 is made, into which the loose products P are introduced, coming from the loading side 24 and comprising said helicoidal guide 48, able to guide and move the loose products P from the loading side 24 to the unloading side 26, during the rotation of the container 20.

The helicoidal guide 48 can provide suitably shaped and distanced spirals 36, which can be in the form of a screw, a worm screw or suchlike, so as to suitably guide the loose products P from the loading side 24 to the unloading side 26 of the container 20.

Downstream of the unloading side 26 of the container 20, in particular downstream of the tubular transfer element 29, a metering device 43 is positioned, for example a metering cylinder, suitable to house a certain quantity of loose products P.

For example, it can be hypothesized that the quantity of loose products P intended to be poured into a determinate containing sector 15 of the drum 12 is introduced into the metering device 43, depending for example on the type of loose products P, the treatment cycle to be carried out or other.

The sterilizing machine 10 also comprises a pneumatic device 44 to send, in particular automatically, the loose products P coming from the container 20 toward the loading zone 14 of the treatment chamber 11.

The sending or transfer of loose products P can be performed by means of a suitable transfer pipe 19, for example a flexible pipe 19.

The pneumatic device 44 can be, for example, a blower or suchlike, which can be associated with a connector 45, which in turn can be connected to the pipe 19 and to an outlet tubular element 46 of the metering device 43.

The pipe 19, therefore, can be connected at one end to the connector 45 and, at the other end, to the aperture 30 of the loading zone 14 of the treatment chamber 11.

A sealing gasket, for example a pressurized gasket or suchlike, can be positioned in correspondence with the loading aperture 30.

In the present sterilizing machine 10, the loose products P are therefore loaded automatically and precisely by means of the loading container 20, which keeps the loose products P in motion and allows a suitable transfer thereof from the loading side 24 to the unloading side 26.

The loose products P to be treated can be loaded from the loading side 24 into the container 20 by means of the hopper 40.

Once the desired loading of the loose products P has been completed, the container 20 can be made to rotate around the axis C, by means of the drive motor 38, so as to keep the loose products P contained in the internal chamber 35 in motion.

By means of the helicoidal guide 48, the loose products P are suitably guided and moved from the loading side 24 to the unloading side 26, from which they can exit through the tubular element 27.

It is possible to provide that the loose products P are transferred directly into the pipe 19 and that the pneumatic device 44 can automatically transfer a predetermined quantity of loose products P to the loading zone 14 of the treatment chamber 11, by means of the pipe 19, for example by providing that the valve 31 closes after a certain quantity of loose products P has passed to the tubular element 29.

The valve 31 to open and close the passage of loose products P could cooperate with the control device 39 located in the loading zone 14, so that, for example, it is closed when the control device 39 detects that the containing sector 15 located in correspondence with the loading zone 14 is filled with the desired quantity of loose products P.

However, preferably, it is provided that the loose products P are transferred to the metering device 43, by means of which it is possible to establish the correct quantity of loose products to be sent into each of the containing sectors 15 of the drum 12.

Substantially then, once the desired quantity of loose products P is present in the metering device 43, the pneumatic device 44 will be driven so as to automatically transfer the loose products P to the aperture 30 of the loading zone 14 of the treatment chamber 11, along the pipe 19.

In this step, the valve 31 will be closed, so as to prevent other loose products from reaching the metering device 43.

Naturally, in this step, the valve 22 positioned in the loading zone 14 will be kept open and the quantity of loose products P will be introduced into a determinate containing sector 15, which presents itself with the door 37 open in correspondence with the loading aperture 30.

Once the loading of the loose products P into the containing sector 15 has been completed, the door 37 is closed, the drum 12 rotated so as to present another containing sector 15 in correspondence with the loading zone, with the corresponding door 37 open.

As we said above, once one containing sector 15 has been loaded, the control device 39 can automatically send a "loading completed" signal to the control unit 47 of the sterilizing machine 10, so that, for example, it opens the valve 31 again and activates a new unloading of loose products P to the metering device 43.

Advantageously, it is possible to provide that the control unit 47 is connected at least to the valve 31, the metering device 43 and the control device 39.

Then, a new transfer of loose products P from the container 20 to the metering device 43 is carried out, so as to be able to send another quantity of loose products P to the other containing sector 15, which is now located in correspondence with the loading zone 14.

Therefore, the transfer of loose products P from the container 20 to the treatment chamber 11 is carried out, according to the methods described above, until all the containing sectors 15 of the drum are filled with the desired quantity of loose products P to be treated.

As can be understood, the transfer of the loose products P from the container 20 to the pneumatic device 44, and then to the loading zone 14 of the treatment chamber 11, takes place in a very uniform, efficient and precise manner, thanks to the movement to which they are subjected inside the container 20, and therefore thanks in particular to the rotation of the container 20.

Once the loading of the containing sectors 15 of the drum 12 has been completed, the valve 22 is closed by a suitable and automatic translation of the shutter 18, and the sterilization cycle can begin in the treatment chamber 11.

Once the treatment cycle is completed, the loose products P are unloaded from the containing sectors 15 to the unloading aperture 16 of the treatment chamber 11, where they can be collected in suitable containers.

As we said, the control device 39 can monitor the operations of the treatment cycle both during the loading step of the loose products P to be subjected to the treatment cycle, and also at the end of the cycle, so as to verify, for example, that possible loose products P treated have not been undesirably trapped inside the containing sectors 15.

This control, as mentioned in the preamble, is important, as it allows to avoid mixing loose products P to be treated with loose products treated, and also to avoid mixing loose products P of different types and/or belonging to different treatment batches.

Advantageously, in the present sterilizing machine 10, this control takes place automatically thanks to the control device 39.

## Claims

1. Sterilizing machine for loose products (P), comprising a treatment chamber (11) provided with a loading zone (14) for said loose products (P) to be treated, said sterilizing machine being **characterized in that** it comprises, upstream of said treatment chamber (11):
a container (20) for loading said loose products (P) provided with a loading side (24) and an unloading side (26) of said loose products (P), said container (20) being made to rotate by means of a drive motor (38) around an axis (C) that passes through said loading side (24) and said unloading side (26);
at least one helicoidal guide (48) with several spirals (36), positioned inside said container (20) and configured to move and guide said loose products (P) from said loading side (24) to said unloading side (26),
at least a pipe (19) for sending the loose products (P) from said container (20) to said treatment chamber (11); and
at least one pneumatic device (44) positioned on the unloading side (26) of said container (20) and configured to introduce said loose products (P) unloaded from said container (20) into said pipe (19) and hence toward said loading zone (14) of said treatment chamber (11).

2. Machine as in claim 1, **characterized in that** said container (20) is a rotatable drum provided with a cylindrical casing (32) comprising an internal chamber (35) where said helicoidal guide (48) develops along said axis (C) of rotation.

3. Machine as in claim 1 or 2, **characterized in that** said container (20) comprises on the loading side (24) a first tubular element (25) communicating with a hopper (40) to load the loose products (P) and, on the unloading side (26), a second tubular element (27) to transfer the loose products (P) toward said pneumatic device (44).

4. Machine as in claim 3, **characterized in that** said container (20) comprises truncated cone parts (33, 34) to connect with said tubular elements (25, 27).

5. Machine as in claim 3, **characterized in that** said first tubular element (25) is integrated with said rotating container (20) and is connected to said loading hopper (40) by means of a rotatable joint (21).

6. Machine as in claim 3, **characterized in that** said second tubular element (27) is integrated with said rotating container (20) and is connected, by means of a rotatable joint (28), to another tubular element (29) to transfer the loose products (P).

7. Machine as in any claim hereinbefore, **characterized in that** it comprises a valve (31) for opening and closing the passage of the loose products (P) positioned downstream of the unloading side (26) of said container (20) and upstream of said pneumatic device (44).

8. Machine as in any claim hereinbefore, **characterized in that** it comprises a metering device (43) to meter said loose products (P), positioned downstream of said loading container (20).

9. Machine as in claim 8, **characterized in that** said metering device (43) is positioned upstream of a connector (45) connecting with said pneumatic device (44) for sending said loose products (P) to said treatment chamber (11).

10. Machine as in any claim hereinbefore, **characterized in that** it comprises a control device (39) inside said treatment chamber (11).

11. Machine as in claims 7, 8 and 10, **characterized in that** it comprises at least a control unit (47) connected at least to said valve (31), to said metering device (43) and to said control device (39).

12. Machine as in any claim hereinbefore, **characterized in that** it comprises a frame (41) provided with rolling elements (42) to position and support the rotatable container (20).

13. Machine as in any claim hereinbefore, **characterized in that** said container (20) has an inclination from the top downward from said loading side (24) to said unloading side (26).

14. Method for sterilizing loose products (P), carried out in a sterilizing machine (10) for said loose products (P) as in any claim hereinbefore, said method being **characterized in that** it comprises loading said loose products (P) to be treated into a loading container (20) provided with a loading side (24) and an unloading side (26) and rotatable by means of a drive motor (38) around an axis (C) that passes through said loading side (24) and said unloading side (26); guiding and moving said loose products by means of at least a helicoidal guide (48) with several spirals (36) positioned inside said container (20) and configured to guide and move said loose products (P) from said loading side (24) to said unloading side (26); and sending said loose products (P) toward a loading zone (14) of a treatment chamber (11) of said sterilizing machine (10) once said loose products (P) have exited from said unloading side (26) of said loading container (20), said sending being carried out by means of a pneumatic device (44) that introduces the loose products (P) exiting from said unloading side (26) into a pipe (19) provided between the loading side (24) of the treatment chamber (11) and said unloading side (26) of the container (20).

## Patentansprüche

1. Sterilisierungsmaschine für lose Produkte (P), aufweisend eine Behandlungskammer (11), die mit einer Ladezone (14) für die zu behandelnden losen Produkte (P) bereitgestellt ist, wobei die Sterilisierungsmaschine **dadurch gekennzeichnet ist, dass** sie stromaufwärts von der Behandlungskammer (11) aufweist:
einen Behälter (20) zum Laden der losen Produkte (P), der mit einer Laden-Seite (24) und einer Entladen-Seite (26) der losen Produkte (P) bereitgestellt ist, wobei der Behälter (20) gemacht ist, um mittels eines Antriebsmotors (38) um eine Achse (C) zu rotieren, die durch die Laden-Seite (24) und die Entladen-Seite (26) hindurch verläuft,
mindestens eine wendelförmige Führung (48) mit mehreren Wendeln (36), die im Inneren des Behälters (20) positioniert ist und eingerichtet ist, um die losen Produkte (P) von der Laden-Seite (24) zur Entladen-Seite (26) zu bewegen und zu führen,
mindestens ein Rohr (19) zum Befördern der losen Produkte (P) vom Behälter (20) zur Behandlungskammer (11), und
mindestens eine pneumatische Vorrichtung (44), die an der Entladen-Seite (26) des Behälters (20) positioniert ist und eingerichtet ist, um die aus dem Behälter (20) entladenen losen Produkte (P) in das Rohr (19) und damit zu der Ladezone (14) der Behandlungskammer (11) hin einzuführen.

2. Maschine nach Anspruch 1, **dadurch gekennzeichnet, dass** der Behälter (20) eine drehbare Trommel ist, die mit einem zylinderförmigen Gehäuse (32) ausgestattet ist, das eine innere Kammer (35) aufweist, in der sich die wendelförmige Führung (48) entlang der Rotationsachse (C) erstreckt.

3. Maschine nach Anspruch 1 oder 2, **dadurch gekennzeichnet, dass** der Behälter (20) aufweist auf der Laden-Seite (24) ein erstes rohrförmiges Element (25), das mit einem Trichter (40) in Verbindung steht, um die losen Produkte (P) zu laden, und auf der Entladen-Seite (26) ein zweites rohrförmiges Element (27), um die losen Produkte (P) zu der pneumatischen Vorrichtung (44) hin zu transferieren.

4. Maschine nach Anspruch 3, **dadurch gekennzeichnet, dass** der Behälter (20) kegelstumpfartige Konusteile (33, 34) zur Verbindung mit den rohrförmigen Elementen (25, 27) aufweist.

5. Maschine nach Anspruch 3, **dadurch gekennzeichnet, dass** das erste rohrförmige Element (25) mit dem drehenden Behälter (20) integral ist und mittels einer Drehverbindung (21) mit dem Ladetrichter (40) verbunden ist.

6. Maschine nach Anspruch 3, **dadurch gekennzeichnet, dass** das zweite rohrförmige Element (27) mit dem drehenden Behälter (20) integral ist und mittels einer Drehverbindung (28) mit dem anderen rohrförmigen Element (29) verbunden ist, um die losen Produkte (P) zu transferieren.

7. Maschine nach irgendeinem vorhergehenden Anspruch, **dadurch gekennzeichnet, dass** sie ein Ventil (31) zum Öffnen und Schließen des Durchgangs der losen Produkte (P) aufweist, das stromabwärts von der Entladen-Seite (26) des Behälters (20) und stromaufwärts von der pneumatischen Vorrichtung (44) positioniert ist.

8. Maschine nach irgendeinem vorhergehenden Anspruch, **dadurch gekennzeichnet, dass** sie eine Zählvorrichtung (43) zum Zählen der losen Produkte (P) aufweist, die stromabwärts von dem Ladebehälter (20) positioniert ist.

9. Maschine nach Anspruch 8, **dadurch gekennzeichnet, dass** die Zählvorrichtung (43) stromaufwärts von einem Verbinder (45) positioniert ist, der mit der pneumatischen Vorrichtung (44) zum Befördern der losen Produkte (P) zur Behandlungskammer (11) verbunden ist.

10. Maschine nach irgendeinem vorhergehenden Anspruch, **dadurch gekennzeichnet, dass** sie eine Steuervorrichtung (39) im Inneren der Behandlungskammer (11) aufweist.

11. Maschine nach den Ansprüchen 7, 8 und 10, **dadurch gekennzeichnet, dass** sie mindestens eine Steuereinheit (47) aufweist, die mindestens mit dem Ventil (31), mit der Zählvorrichtung (43) und mit der Steuervorrichtung (39) verbunden ist.

12. Maschine nach irgendeinem vorhergehenden Anspruch, **dadurch gekennzeichnet, dass** sie einen Rahmen (41) aufweist, der mit Rollelementen (42) bereitgestellt ist, um den drehbaren Behälter (20) zu positionieren und zu halten.

13. Maschine nach irgendeinem vorhergehenden Anspruch, **dadurch gekennzeichnet, dass** der Behälter (20) eine Neigung von oben nach unten von der Laden-Seite (24) aus zur Entladen-Seite (26) hat.

14. Verfahren zum Sterilisieren loser Produkte (P), das in einer Sterilisierungsmaschine (10) für lose Produkte (P) nach irgendeinem vorhergehenden Anspruch durchgeführt wird, wobei das Verfahren **dadurch gekennzeichnet ist, dass** es aufweist Laden zu behandelnder loser Produkte (P) in einen Ladebehälter (20), der mit einer Laden-Seite (24) und einer Entladen-Seite (26) bereitgestellt ist und mittels eines Antriebsmotors (38) um eine Achse (C) drehbar ist, die durch die Laden-Seite (24) und die Entladen-Seite (26) hindurch verläuft, Führen und Bewegen der losen Produkte mittels mindestens einer wendelförmigen Führung (48) mit mehreren Wendeln (36), die im Inneren des Behälters (20) positioniert ist und eingerichtet ist, um die losen Produkte (P) von der Laden-Seite (24) zur Entladen-Seite (26) zu führen und zu bewegen, und Befördern der losen Produkte (P) zu einer Ladezone (14) einer Behandlungskammer (11) der Sterilisierungsmaschine (10) hin, sobald die losen Produkte (P) aus der Entladen-Seite (26) des Ladebehälters (20) ausgetreten sind, wobei das Befördern mittels einer pneumatischen Vorrichtung (44) durchgeführt wird, die die losen Produkte (P), die aus der Entladen-Seite (26) austreten, in ein Rohr (19) einführt, das zwischen der Laden-Seite (24) der Behandlungskammer (11) und der Entladen-Seite (26) des Behälters (20) bereitgestellt ist.

## Revendications

1. Machine de stérilisation de produits en vrac (P), comprenant une chambre de traitement (11) pourvue d'une zone de chargement (14) pour lesdits produits en vrac (P) à traiter, ladite machine de stérilisation étant **caractérisée en ce qu'**elle comprend, en amont de ladite chambre de traitement (11) :
un conteneur (20) pour charger lesdits produits en vrac (P) pourvu d'un côté de chargement (24) et d'un côté de déchargement (26) desdits produits en vrac (P), ledit conteneur (20) étant amené à tourner au moyen d'un moteur d'entraînement (38) autour d'un axe (C) qui passe à travers ledit côté de chargement (24) et ledit côté de déchargement (26) ;
au moins un guide hélicoïdal (48) avec plusieurs spirales (36), positionné à l'intérieur dudit conteneur (20) et configuré pour déplacer et guider lesdits produits en vrac (P) dudit côté de chargement (24) vers ledit côté de déchargement (26),
au moins un tuyau (19) pour envoyer les produits en vrac (P) dudit conteneur (20) vers ladite chambre de traitement (11) ; et
au moins un dispositif pneumatique (44) positionné sur le côté de déchargement (26) dudit conteneur (20) et configuré pour introduire lesdits produits en vrac (P) déchargés dudit conteneur (20) dans ledit tuyau (19) et donc vers ladite zone de chargement (14) de ladite chambre de traitement (11).

2. Machine selon la revendication 1, **caractérisée en ce que** ledit conteneur (20) est un tambour rotatif muni d'une enveloppe cylindrique (32) comprenant une chambre interne (35) où ledit guide hélicoïdal (48) se développe le long dudit axe de rotation (C).

3. Machine selon la revendication 1 ou 2, **caractérisée en ce que** ledit conteneur (20) comprend sur le côté de chargement (24) un premier élément tubulaire (25) communiquant avec une trémie (40) pour charger les produits en vrac (P) et, sur le côté de déchargement (26), un second élément tubulaire (27) pour transférer les produits en vrac (P) vers ledit dispositif pneumatique (44).

4. Machine selon la revendication 3, **caractérisée en ce que** ledit conteneur (20) comprend des parties tronconiques (33, 34) pour se connecter auxdits éléments tubulaires (25, 27).

5. Machine selon la revendication 3, **caractérisée en ce que** ledit premier élément tubulaire (25) est intégré audit conteneur rotatif (20) et est connecté à ladite trémie de chargement (40) au moyen d'un joint rotatif (21).

6. Machine selon la revendication 3, **caractérisée en ce que** ledit second élément tubulaire (27) est intégré audit conteneur rotatif (20) et est connecté au moyen d'un joint rotatif (28) à un autre élément tubulaire (29) pour transférer les produits en vrac (P).

7. Machine selon l'une quelconque des revendications précédentes, **caractérisée en ce qu'**elle comprend une vanne (31) pour ouvrir et fermer le passage desdits produits en vrac (P) positionnée en aval du côté de déchargement (26) dudit conteneur (20) et en amont dudit dispositif pneumatique (44).

8. Machine selon l'une quelconque des revendications précédentes, **caractérisée en ce qu'**elle comprend un dispositif de dosage (43) pour doser lesdits produits en vrac (P), positionné en aval dudit conteneur de chargement (20).

9. Machine selon la revendication 8, **caractérisée en ce que** ledit dispositif de dosage (43) est positionné en amont d'un connecteur (45) se connectant audit dispositif pneumatique (44) pour envoyer lesdits produits en vrac (P) vers ladite chambre de traitement (11).

10. Machine selon l'une quelconque des revendications précédentes, **caractérisée en ce qu'**elle comprend un dispositif de commande (39) à l'intérieur de ladite chambre de traitement (11).

11. Machine selon les revendications 7, 8 et 10, **caractérisée en ce qu'**elle comprend au moins une unité de commande (47) connectée au moins à ladite vanne (31), audit dispositif de dosage (43) et audit dispositif de commande (39).

12. Machine selon l'une quelconque des revendications précédentes, **caractérisée en ce qu'**elle comprend un cadre (41) muni d'éléments roulants (42) pour positionner et supporter le conteneur rotatif (20).

13. Machine selon l'une quelconque des revendications précédentes, **caractérisée en ce que** ledit conteneur (20) présente une inclinaison du haut vers le bas depuis ledit côté de chargement (24) vers ledit côté de déchargement (26).

14. Procédé de stérilisation de produits en vrac (P), mis en oeuvre dans une machine de stérilisation (10) desdits produits en vrac (P) selon l'une quelconque des revendications précédentes, ledit procédé étant **caractérisé en ce qu'**il comprend le chargement desdits produits en vrac (P) à traiter dans un conteneur de chargement (20) pourvu d'un côté de chargement (24) et d'un côté de déchargement (26) et pouvant tourner au moyen d'un moteur d'entraînement (38) autour d'un axe (C) qui passe par ledit côté de chargement (24) et ledit côté de déchargement (26) ; le guidage et le déplacement desdits produits en vrac au moyen d'au moins un guide hélicoïdal (48) avec plusieurs spirales (36) positionné à l'intérieur dudit conteneur (20) et configuré pour guider et déplacer lesdits produits en vrac (P) dudit côté de chargement (24) vers ledit côté de déchargement (26) ; et l'envoi desdits produits en vrac (P) vers une zone de chargement (14) d'une chambre de traitement (11) de ladite machine de stérilisation (10) une fois que lesdits produits en vrac (P) sont sortis dudit côté de déchargement (26) dudit conteneur de chargement (20), ledit envoi étant effectué au moyen d'un dispositif pneumatique (44) qui introduit les produits en vrac (P) sortant dudit côté de déchargement (26) dans un tuyau (19) prévu entre le côté de chargement (24) de la chambre de traitement (11) et ledit côté de déchargement (26) du conteneur (20).
